(19) Europäisches Patentamt

European Patent Office

Office européen des brevets

(11) **EP 1 607 881 A2**

(12) **EUROPEAN PATENT APPLICATION**

(43) Date of publication:
21.12.2005 Bulletin 2005/51

(51) Int Cl.⁷: **G06F 17/00**

(21) Application number: 05253798.2

(22) Date of filing: 20.06.2005

| | |
|---|---|
| (84) Designated Contracting States:<br>**AT BE BG CH CY CZ DE DK EE ES FI FR GB GR HU IE IS IT LI LT LU MC NL PL PT RO SE SI SK TR**<br>Designated Extension States:<br>**AL BA HR LV MK YU**<br><br>(30) Priority: **19.06.2004  GB 0413824**<br><br>(71) Applicant: **Dr Foster Limited**<br>**London EC3A 6DG (GB)** | (72) Inventors:<br>• **Taylor, Roger**<br>**London EC3A 6DG (GB)**<br>• **Middleton, Steven**<br>**London EC3A 6DG (GB)**<br><br>(74) Representative: **Mackenzie, Andrew Bryan et al**<br>**Marks & Clerk**<br>**45 Grosvenor Road**<br>**St. Albans, Hertfordshire AL1 3AW (GB)** |

(54) **System for data analysis**

(57)     A method and apparatus for analysing data allowing accurate, up to date analysis of the performance of hospitals or hospital trusts as the data is entered into the system. The method and apparatus is optimised for analysing data in such a way as to produce graphical representations allowing easy recognition of groups of patient having or hospitals producing outcomes which have significantly diverged from the desired outcome. The method involves filtering data held within databases to retrieve data belonging to the patient group that is to be analysed. The filtered data is then analysed using statistical calculations and a representation of the analysis is returned to the user for review.

Figure 1

**Description**

This invention relates to a methods and apparatus for analysing data. The invention is particularly applicable to monitoring performance within a Patient Records System.

In the United Kingdom performance monitoring is carried out using annual data collated since 1996. The data is collated from the national Hospital Episode Statistics (HES) dataset. The HES dataset is collated using data received from all English NHS hospital trusts. In the UK, hospitals are managed firstly on an individual level and then at a trust level, therefore, a hospital trust is a set of hospitals having the same controlling management team. The data held within the HES dataset is based on periods of treatment such as a stay in hospital known as episodes. Episodes may be linked together into "spells" representing a continuous period of care for a patient within an NHS trust, and further into superspells using data from other NHS trusts in which the patient was treated, for example by being transferred. This linking is done using fuzzy logic based on the age, sex, postcode and date of discharge of each patient. Hence, a superspell defines a continuous period of care across a number of NHS trusts (for a single period of illness for a patient).

Further data may be retrieved from another dataset known as the national NHS-Wide Clearing Service (NWCS) dataset which is updated monthly. Data for this dataset is linked using the same fuzzy logic as is used with the annual HES dataset. However, currently there is no integrated mechanism for quick and efficient statistical analysis of the data contained within these databases. The data is currently only collated and analysed for reports and performance tables resulting in slow reactions within hospitals when their performance falls significantly below the mean. The current invention provides a mechanism allowing data to be analysed within a minimal amount of time, thereby allowing hospitals and their staff to react quickly to redress any fall in performance, helping to minimise any danger to patient safety.

According to a first aspect of the invention there is provided a method for and apparatus adapted for analysing data comprising the steps of: receiving patient data; receiving criteria representative of the patient data to be analysed; filtering the patient data according to the criteria; and calculating a representation of the filtered data. This provides the advantage that data is quickly analysed and presented to users of the system, allowing them to easily pinpoint any problems identified within the data.

Preferably the method is used to produce a control chart and the control chart is preferably, calculated using patient data that is weighted according to the patient outcome.

Preferably the method also sets a threshold value above which the graph has diverged significantly from a benchmark value. This allows a user to easily and quickly identify when performance has become poor enough to require measures to be taken to bring it back towards the benchmark value.

Preferably the method includes receiving an option according to which the patient data is to be grouped; and grouping the patient data according to the analysis option. This allows easy comparisons between equivalent patient groups in different hospitals or trusts, or different patient groups within the same trust. This allows bodies, such as government, to know where and what are causing different negative outcomes within the hospitals, helping them tackle any causes of poor outcome rates.

Embodiments of the invention will now be described, by way of example, and with reference to the drawings in which:

Figure 1 illustrates the way in which the data analysis is carried out;

Figure 2 illustrates a CUSUM chart representation of the filtered data;

Figure 3 illustrates a representation of alerts that have been signalled;

Figure 4a illustrates a visual representation of relative risk data; and

Figure 4b illustrates an alternative representation of relative risk data.

The following describes the dataset which is currently being used by the system. It is provided as an example and to aid clarity when describing the system's functionality. The system may also be used to measure different outcomes or use similar data from other sources.

The system applies statistical process control methodology to health outcomes within hospitals. Control charts such as cumulative sum (CUSUM) charts are used to determine if the outcomes have diverged significantly from a benchmark value. The benchmark values are derived from national dataset and are preferably equal to the average outcome for a relevant group of patients.

Preferably, the system uses real-time or near-real-time data in order to provide users of the system with up to date data. The use of real-time or near-real-time data allows the users to quickly determine when outcomes have diverged significantly from a benchmark value in order to allow appropriate corrective measures to be taken promptly. Furthermore, the system may be configured to alert users to outcomes that have diverged significantly from the mean. Users may also generate CUSUM charts and other analyses at various levels of the health organisation in order to assist the process of internal auditing.

CUSUM

The data held in the national databases may be used to generate control charts, such as cumulative sum (CUSUM) charts, alerts and associated analyses. Control charts are designed to highlight significant variation within a dataset. Generally, they have a line demarking the mean number of outcomes (equivalent to our benchmark) and statistical control limits (equivalent to our threshold). The statistical control limits mark the point at which the data may be considered to have diverged to too great an extent from the mean value. Therefore, these limits highlight the point at which action needs to be taken to bring the chart closer to the mean. Suitable chart types include Shewhart-type charts and CUSUM charts.

Preferably the system is optimised to generate CUSUM charts. The CUSUM chart may either be generated by the selection of criteria and CUSUM parameters or the selection of an Alert. Alerts have predefined criteria and parameters that are associated with them and when selected the Alerts generate the CUSUM chart associated with their associated criteria and parameters.

Criteria may include:

The trust or hospital responsible for the care of the patient. These are selected according to the first episode and only the superspells of patients treated within the selected trust or hospital are analysed. Preferably only users having access to the data of multiple trusts may select to view the data of a trust. For example, the user may be a Strategic Health Authority which is responsible for co-ordinating health care over a region.

Whether the data for analysis should be filtered in terms of diagnoses or procedures. Preferably the division of diagnoses and procedures is according to: Emergency diagnoses, the diagnosis groups accounting for 80% of all in-hospital mortaility and Surgical Procedures. For Emergency Diagnoses, the primary diagnosis of the first episode of the first spell determines which sub group a superspell is assigned to. The admission, however, must also have been an emergency. For Top 80 diagnoses, the primary diagnosis of the first episode determines which of the diagnosis groups accounting for 80% of all in-hospital mortality a superspell is assigned to. Finally if a surgical procedure has been performed, each superspell is assigned to a group based on the main operation in the first episode of the first spell. Procedure groups may be further sub-divided according to the method of admission, primary diagnosis and other criteria. Preferably a selection of one of the relevant groups is made.

Further sub-division or aggregation of these groups into chapters is also possible. Preferably this is done using the first letter of the codes (ICD10/OPCS4 when selecting diagnoses or procedures respectively) assigned to the diagnosis or procedure, known as a chapter. Preferably all relevant diagnoses or procedures are selected as a default.

The sub-division may be further narrowed down to a specific diagnosis or procedure which are included in the chosen diagnosis or procedure and the selected sub-division. Preferably all relevant diagnoses or procedures are selected as a default.

Alternatively, the diagnosis or procedure group may be narrowed according to the relevant specialty. This allows the user to aggregate consultant teams or narrow down the list of consultant teams from which to select. Preferably the unit administrator defines the specialities locally. Preferably all relevant diagnoses or procedures are selected as a default.

The consultant team responsible for the care of the patient during the first episode of the superspell may also be selected. Preferably all relevant diagnoses or procedures are selected as a default.

The outcome to be charted may also be selected from a list of outcomes relevant to the selected diagnosis or procedure. The outcomes include: mortality (whether in-hospital or within a specified time period); length of stay (whether it is longer than a specified time period); whether the patient was treated as a day case and whether or not the patient was readmitted within a specified number of days after leaving hospital. Preferably the outcome may be measured in terms of whether it is true or false. Other outcomes may also be defined as required and management or data-quality outcomes may also be monitored. Preferably an outcome to be analysed is selected.

Other factors which may also be selected and have "All" as the default selection are: the patient's age range, gender deprivation quintile (preferably ranging from "affluent" to "deprived"), admission type (whether "emergency" or "elective") and the date range over which the data to be analysed is selected.

The CUSUM parameters include:

The odds ratio ($R_A$). This rate reflects a change in performance that is deemed to have diverged too greatly from the benchmark and is, therefore, interesting. Preferably $R_A$ is set equal to 2, corresponding to a doubling in the probability of negative outcome.

The currently acceptable level of performance ($R_0$), which preferably corresponds to the existing benchmark. The national benchmark value is calculated as an average for each outcome type for every type of patient. It is calculated using the following data: the year of discharge (this may be grouped), admission type (whether emergency, elective or transfer), diagnosis or procedure and the patient's sex, age group (this is preferably selected as a five year range), and deprivation quintile. The benchmark calculation is preferably also dependent upon the patient's month of admission and further sub-divisions of their diagnosis/procedure group.

The resulting benchmark value may then be standardised for the entire patient population. This provides a pre-admission probability of the occurrence of a particular outcome for every type of patient. Alternative benchmarks, such as the national averages of other countries or an individual trust's averages. The benchmarks may also be presented as percentages.

A threshold value, showing when the CUSUM statistic has significantly diverged from the benchmark value. It, preferably, corresponds to the negative outcome rate being equal or greater than the odds ratio x the benchmark and has a default value of 5. Once the threshold value is exceeded an alert may be signalled. The probability of the negative outcome rate being equal or greater than the odds ratio x the benchmark may be shown on the chart as the False Alarm (FAR) and Successful Detection (SAR) rates.

The parameters also allow a user to select whether the CUSUM chart to be calculated is a positive or negative CUSUM chart.

Finally, the user may also be able to determine how the CUSUM statistic is reset after it reaches the threshold value. Preferably, the CUSUM statistic is reset to half the threshold value. However, in order to review the trend in performance over time the user may select not to reset the statistic in which case the chart can continue to rise above the threshold.

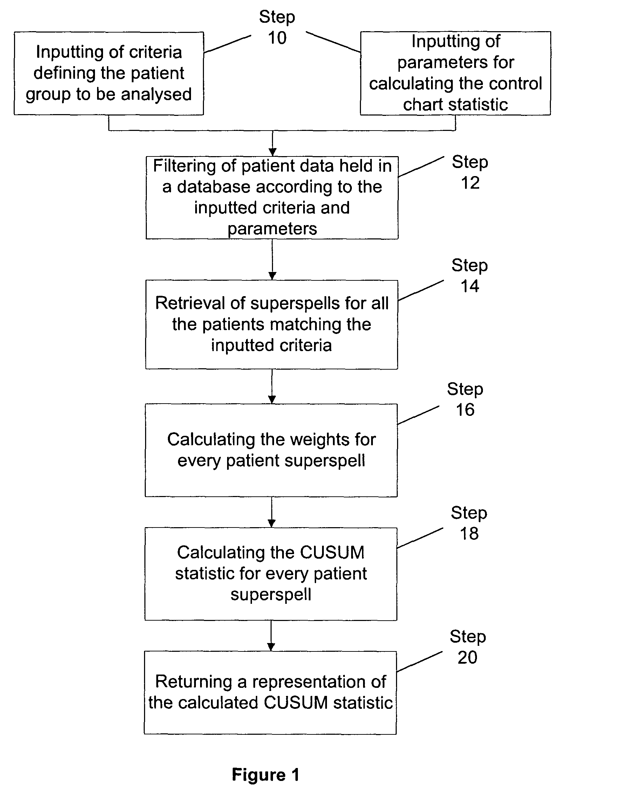

Figure 1 illustrates the method of calculating the CUSUM statistic:

1. In step 10 the user selects patient criteria and CUSUM parameters and inputs them into the system. Preferably the criteria 30 and parameters 32 are selected using dropdown menus in the bottom frame 28 of a webpage window 22 displayed when the "CUSUM" tab is selected. Once selected the criteria and parameters pass to a stored procedure in a database containing the patient data.

2. In step 12 the stored procedure selects all the superspells in the database that match the selected criteria. Additionally, the data may be ordered according to the procedure or admission date preferably with the most recent spell last.

3. In step 14, the observed outcome and the expected outcome (i.e. the benchmark for the patient's type) for every selected superspell are returned to a processor. Preferably the observed and expected outcome values are figures between 0 and 1.

4. In step 16, the processor calculates the "weight" of each superspell according to the formulae:

$$W_t = \log \frac{(1-p_t+R_0 p_t)R_A}{(1-p_t+R_A p_t)R_o}$$

if $y_t=1$ (i.e. if the outcome is negative) and

$$W_t = \log \frac{1-p_t+R_0 p_t}{1-p_t+R_A p_t}$$

if $y_t=0$ (i.e. if the outcome is positive)
where $R_0$ corresponds to the existing benchmark;
$R_A$ corresponds to a change in performance deemed interesting (i.e. unacceptably high);
$y_t$ is the actual patient outcome;
and $p_t$ is the estimated risk, if this is likely to have changed then more recent data may be used for its estimation.

5. In step 18 the processor further calculates a CUSUM chart representation for each superspell using the weights calculated using the formulae above according to the following formulae:

$$X_t = \max(0, X_{t-1} + W_t), t=1, 2, 3...$$

for a negative CUSUM chart

$$X_t = \min(0, X_{t-1} - W_t), t=1, 2, 3...$$

for a positive CUSUM chart
where $X_t$, is the current CUSUM statistic value and $W_t$ is the patient weighting.

The current value, $X_t$, depends on the previous value, $X_{t-1}$, and the patient weight, $W_t$, for patient t.

For a positive CUSUM chart, $R_A$ is set to the reciprocal of the value used for negative CUSUMs. This is preferably 0.5.

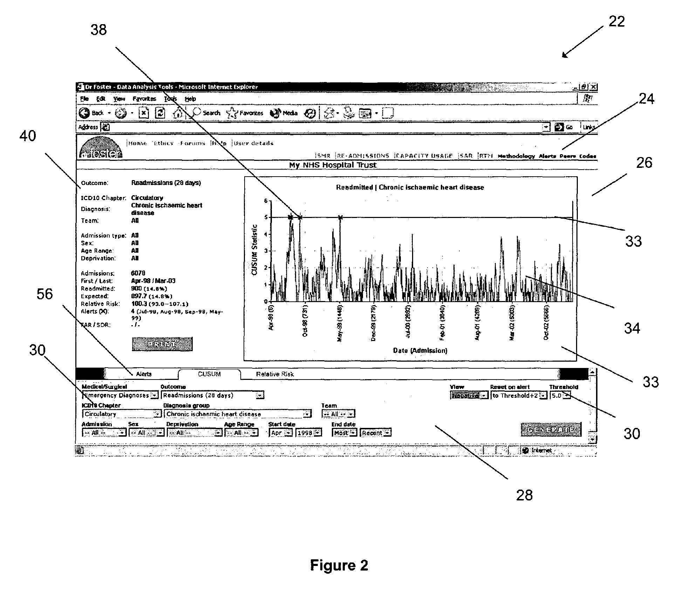

6. In step 20 a representation of the calculated CUSUM statistic is returned to the user. One possible representation of a CUSUM chart is shown in Figure 2. In Figure 2, the CUSUM graph is constructed on a web page 22 with the "patient" or date on the x-axis and the CUSUM statistic on the y-axis. When a "negative" view is selected, the CUSUM representation 34 may plotted in red. When a "positive" view is chosen, the CUSUM representation 34, which would normally have negative values, may be plotted on the positive y-axis and may additionally be coloured green. The threshold 36 may be displayed as a horizontal line where y=threshold value.

7. An alert 38 is set for each superspell where the CUSUM statistic exceeds a chosen threshold value 36. As previously discussed, preferably when the CUSUM statistic reaches the threshold value 36 it is reset to half the threshold value prior to calculating the value for the next superspell. The user may also choose not to reset the CUSUM statistic. An alert 38 may be displayed on the CUSUM chart representation as a black cross on the threshold line.

8. Superspells are preferably grouped by date having the maximum values of the "patient" number, the CUSUM statistic and the number of alerts 38 returned instead. This improves speed by reducing the number of points to be plotted and improves legibility when reading the graph.

9. The total number of superspells, the dates of the first and last superspells, the sum of all the "observed" values, the sum of all the "expected" values and the False Alarm and Successful Detection rates (discussed below) may also be returned as summary data 40 to the web page.

10. Some summary data may also be included on the web page. These include:

The criteria 30 and CUSUM parameters 32 selected and used to generate the CUSUM chart. The total number of superspells matching the selected criteria. The first and last superspell's admission or operation date, for diagnoses or procedures respectively.

The sum of all the "observed" outcome values among the superspells matching the selected criteria. This figure may also be shown as a proportion of the total number of superspells matching the criteria. Preferably the sum of all "observed" values for the outcome matching the patient criteria is shown with a description describing the selected negative outcome.

The sum of all the "expected" outcome value for the superspells matching the selected criteria, which take into account the patient-mix of the selection. The figure may also be shown as a proportion of the total number of superspells matching the criteria.

The ratio of the "observed" outcomes / the "expected" negative outcomes x 100, known as the Relative Risk Ratio (RRR) and provides a measure of risk relative to the benchmark, the 95% confidence limits for the RRR may also be shown.
The 95% confidence limits may be calculated using Byar's approximation.

The observed average length of stay in days for the first spell in each of the superspells that match the criteria. This is compared with the expected average length of stay which is calculated by applying the average length of stay for England, adjusted for the set of patient criteria appropriate to each superspell. The average of these values is then taken. Similar figures are provided for total length of stay which take account of all the spells in each superspell.

The number of alerts 38 that have occurred, the dates of the alerts and the false alarm and successful detection rates.

## False Alarm and Successful Detection rates

The false alarm rate (FAR) is the probability that for a given threshold 36 and negative outcome rate, an alert may be a false alarm. The successful detection rate (SDR) is the probability that a situation where the performance has diverged significantly from the mean will lead to an alert. With real trust data it is impossible to distinguish between a signal due to a genuinely high rate and one due to an "unlucky bad run". Therefore, the FAR and SDR are preferably calculated by simulation as described below. Through simulation it is possible to determine the probability of a signal due to a genuinely high rate and one due to an "unlucky bad run" for a given threshold 38 as the real rate is pre-defined.

Every outcome has two alternatives and, therefore, the patient may be treated as a coin with respect to the outcome. The probability either alternative outcome is 50%. With a coin the number of tails occur according to a binomial distribution or, for just one outcome, a Bernoulli distribution. However, probability of death after surgery for different patient groups ranges from less than 1 % to nearly 50%.

In the simulation artificial hospitals are generated. The hospitals are grouped into "simulation batches" each of which are allocated a constant death rate of p%. However, hospitals within the simulation batch are allocated different p values, for example, p may be equal to 1%, 2%, 3%, 4%, 5% and then graduate in 5% intervals between 5% and 50%. In this way p covers all the death rates currently seen with the procedures and diagnoses currently being analysed.

If, the analysis relates to heart failure, which has a death rate of 20%, data is generated with a death rate of 20%. This requires the number of patients generated for each artificial hospital to be varied according to p: for a death rate of 1%, 2,500 patients are required for 25 deaths to be expected (on average), whereas for p=20%, we only need 125 patients to achieve 25 deaths in the long run. Having randomly generated such patients, we run the CUSUM charts for the 5,000 artificial trusts and record what proportion exceed the threshold (using firstly a threshold of 2 and secondly a threshold of 5). In these CUSUMs, the pre-op risks are all set to one value in the sequence above, e.g. 20% for heart failure. Any resulting signals are false alarms because we have fixed the artificial trusts to have in-control data.

The successful detection rate is estimated by generating out-of-control data, for example, patients with an odds ratio of 2. If a patient group has a death rate of 1%, then the generated patients in the simulation are made to have twice the pre-op risk, i.e. 2%. As p increases, however, the odds of death fall behind the probability of death, e.g. for heart failure the in-control rate is 20%, an odds ratio of 2 is equivalent to an out-of-control rate of 33%, not 40%. The pre-op risks are again all set to one value in the above sequence, for example, 20%. However, as the artificial trusts have been fixed to have out-of-control data, any resulting signals are known to be true alarms. Therefore, the proportion of trusts signalling alerts is the successful detection rate.

These FAR and SDR may be published with the CUSUM chart in order to give the user an idea of the combined effect of the threshold 38 they have chosen and the negative outcome rate for the patient criteria that they have selected. This allows the user to have an understanding of the trade-off required between these two measures (for a given negative outcome rate, the higher the threshold the lower the FAR and the higher the SDR).

The FAR and SDR for a particular set of criteria may be calculated at the same time as the CUSUM statistic using the following method:

1. From the simulations outlined above, the FAR and SDR rates for a wide range of combinations of negative outcome rate and threshold are stored in a database.

2. For every outcome and patient-type (defined by a unique combination of sex, age, admission type, deprivation quintile and their diagnosis/procedure group), the total number of admissions and the total number of observed negative outcomes are calculated for a specified period of time prior to the date of analysis. Preferably the time period is a year. The results of the calculations are stored in the database.

3. The outcomes for the different patient-types matching the selected criteria are summed to give equivalent figures for admissions and observed negative outcomes. From these two figures the national negative outcome rate can be calculated.

4. The closest corresponding FAR and SDR to the calculated outcome rate and selected threshold are selected.

5. The selected FAR and SDR displayed along with the other CUSUM data.

## Alerts

An alert 38 is calculated using a pre-defined set of patient criteria 30 and CUSUM parameters 32. The criteria and parameters are used to automatically generate the CUSUM chart associated with the alert 38. If a chart reaches the threshold value 36 at any point, an alert 38 is said to have signalled. An alert 38 provides an early warning that events are diverging significantly from the benchmark. The divergence may either be negative, due to poor performance, or positive, due to good performance.

Criteria used to calculate an alert 38 may be defined by the system administrator and are used to set a common standard across all trusts. Preferably, they are set for every combination of trust, outcome and groups of diagnoses and procedures. They are also calculated for each consultant team within each trust for which the particular diagnosis or procedure is applicable.

Preferably all system alerts use the national benchmark and the same CUSUM parameters for start date, threshold, odds ratio and $R_0$ value. A high threshold implying a low false alarm rate is also preferably used.

Alternatively alerts may be defined by individual users, these are typically used to set custom targets to monitor local performance. The user has complete control over the criteria 30 chosen and the CUSUM parameters 32 used, subject to any restrictions associated with their level of access.

Whenever new patient data is added to the database, all of the alerts 38 are recalculated. The recalculation uses the same method as described above, except that the criteria 30 and parameters 32 are pre-defined and not selected by a user. The criteria 30 and parameters 32 are preferably stored within a database. Additionally, both negative and positive CUSUM statistics 34 are calculated, there is no date selection and the CUSUM statistic 34 always resets to "Threshold / 2" on reaching the threshold.

Preferably, the number of negative signals, the number of positive signals, the date of the most recent negative signal, the date of the most recent positive signal, the number of admissions, the number of observed negative outcomes and the number of expected negative outcomes for each alert which signals is stored in the database.

Preferably, the user is presented with a summary table 42 of the alerts 38 signalled within a selected time period. The user may be able to select the time period over which they wish to view the number of alerts. The selection may be of all alerts signalled since the beginning of collation of data, this may be retrieved from the database, or, alternatively, only alerts signalled within the most recent N months. For example N may be 1, 3, or 12.

A user may be restricted to only viewing a limited number of alerts 38 in the summary table 42 according to access restrictions associated with their login.

Also presented may be: the consultant team to which the alert applies, or "ALL" if the alert applies to the whole trust; a description of the criteria used when calculating the alert; the number of admissions and the number of negative outcomes observed and expected for the selected criteria and period.

Finally, Relative Risk Ratio (RRR) 44 may also be displayed in the summary table 42. The RRR 44 is calculated as the "observed" number of negative outcomes / the "expected" number of negative outcomes x 100. The 95% confidence limits 46 of the RRR 44 may also be displayed. The limits 46 may be calculated by any suitable means including using Byar's approximation. In Byar's approximation the lower and upper 95% confidence limits are given using the following equations respectively:

$$LowerLimit = \frac{x}{e} \times \left( 1 - \frac{1}{9x} - \frac{1.96}{3\sqrt{x}} \right)^3$$

$$UpperLimit = \frac{(x+1)}{e} \times \left( 1 - \frac{1}{9(x+1)} - \frac{1.96}{3\sqrt{(x+1)}} \right)^3$$

where x is the observed number of events
and e is the expected number of events

Preferably, the RRR 44 is displayed in a larger font and highlighted in red when the lower confidence limit is greater than 100 i.e. is significantly higher than the benchmark value. It is highlighted in green when the upper confidence limit (see below) is less than 100 i.e. is significantly lower than the benchmark and therefore is positive performance.

An icon 48 may displayed indicating whether the alert is negative (poor performance) or positive (good performance). Preferably the icon 48 is a red (for negative alerts) or green (for positive alerts) alarm bell. Alternatively, a value may be displayed indicating the number of negative and positive alerts since the start date. The value may be red (for negative alerts) or green (for positive alerts)

The icons 48 or numbers may be selected by a user in order for that user to view the CUSUM chart having the same criteria as that which signalled the alert.

**Relative Risk Ratio (RRR)**

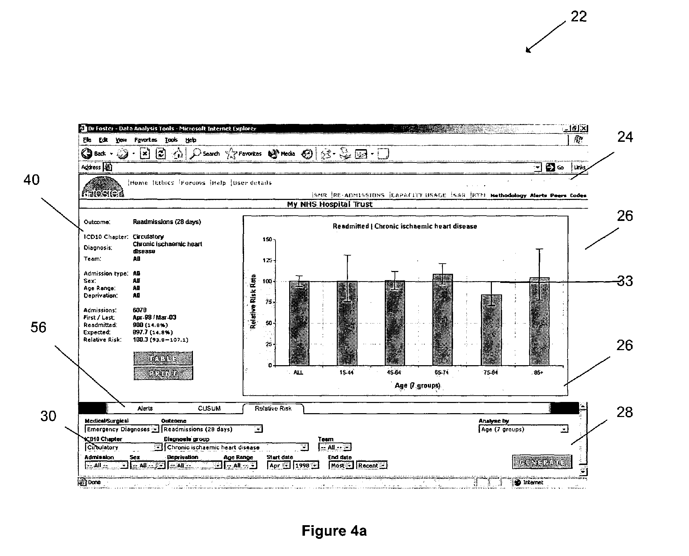

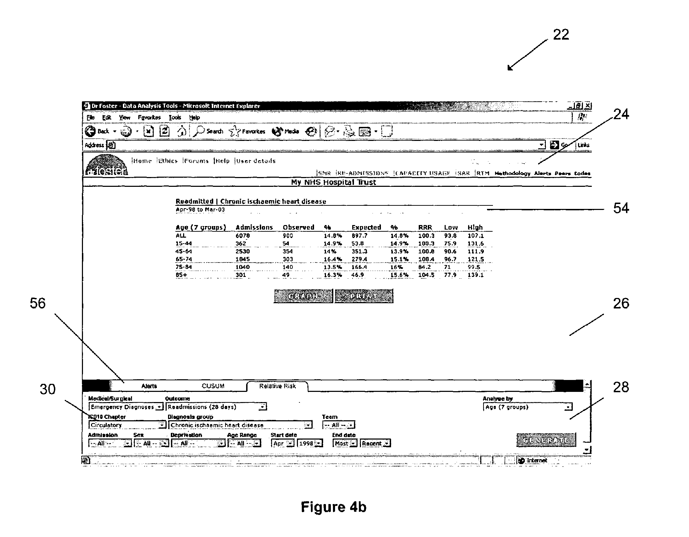

The RRR 44 provides a measure of risk relative to the benchmark for the selected criteria. The representations possible for the RRR 44 are illustrated in Figures 4a and 4b.

The menus for selection of criteria 30 and analysis options 50, described below, may be accessed via a "Relative Risk" tab in the bottom frame 28. The criteria 30 are the same as those described with respect to CUSUM charts. However, the data is further grouped according to options specified by the user. These options include grouping according to the patient's: age group (the entire age spread is preferably divided into 7 or into groups spanning five years), gender, deprivation quintile, GP practice, Electoral ward, Locale or Country of residence, the patient's primary diagnosis or the main surgical procedure carried out, the first letter of the diagnosis or procedure code (ICD10/OPCS4), the speciality, the consultant team, the type of admission (whether emergency or elective), hospital site, the referring PCT, length of stay (either including or excluding transfers), episodes or outcome (for example whether the patient was discharged home, to another hospital or died).

The data is grouped according to the selected analysis option 50 and criteria 30 to produce a graph 52 or a table 54 that displays how the RRR 44 varies according to the analysis option 50 selected. Preferably, this is initiated by the selection of a button.

In addition comparison can be made with a similarly defined set of patients in other trusts. Via the "Peers" option in the top frame 24, users can select peer trusts against which their trust results will be compared. Preferably, this is supplied as an analysis option 50 and when the analysis option 50 is selected the option results in the RRRs 44 of the user-defined peer trusts being displayed.

In the same way, comparison can be made with the, preferably 6, trusts which show either the best performance or the worst performance for a set of patients having the same patient criteria. The best or worst performing trusts are determined by calculating a trust's performance over the period of monitoring specified by the user. The best or worst performing trusts must also have at least half the number of patients matching the selected criteria over this period, as the trust being analysed in order to be compared with the trust being analysed.

Comparison can also be made with the, preferably 6, trusts which have the most similar groups of patient criteria to the trust being analysed. The trusts with the most similar admission criteria are determined by comparing either a trust's percentage admissions, total admissions, or admissions according to groups of patient criteria to the equivalent admissions value of the trust being analysed. The trusts having the smallest squared difference in the equivalent admissions values to those of the trust being analysed are selected to be compared with the trust being analysed.

Preferably users able to view data from multiple trusts, such as Strategic Health Authorities, are provided with an extra analysis option 50 allowing them to compare the RRR 44 of all their trusts.

By selecting Year (Financial), Year (Calendar) or Quarter (Calendar) in the "Analyse by" dropdown, the user can view how the RRR 44 has varied over time.

Relative risk values are calculated using the following method:

1. The selected criteria 30 and analysis option 50 is passed to a stored procedure in the database.

2. The stored procedure selects all the superspells in the database matching the selected criteria 30. For surgical procedures, the date of the procedure is used to match to the start and end dates, otherwise it is the admission date.

3. For every superspell, the observed outcome and the expected outcome values are selected.

4. The superspells are grouped according to the selected analysis option 50, and the total number of superspells, the sum of the "observed" values and the sum of the "expected" values are calculated for each of the groups and for the total.

5. The grouped data, preferably with the dates of the first and last superspells, is returned to the web page. It may then be displayed as either a graph 52 or a table 54.

The user may be able to alternate between the graph 52 and the table 54 by selecting buttons entitled graph or table respectively. In the graph 52 the analysis option groups and total are represented on the x-axis. The RRR for each group and for the total is plotted as a bar on the y-axis. Preferably When "Year" is the selected analysis option the data is plotted as a line rather than a bar. 95% Confidence limits 46 for the RRR 44 may also be displayed as a vertical line on each group bar. The benchmark (100) may also be displayed as a horizontal line.

The selected patient criteria 30 and summary information 40 similar to that shown with the CUSUM chart 33 may also be displayed with the graph 52.

In the table view a summary of the selected criteria 30 is displayed at the top of the table along with the time period over which data was analysed. The "admissions", "observed" negative outcomes, "observed" negative outcome rate, "expected" negative outcomes, "expected" negative outcome rate, relative risk ratio and 95% confidence limits are displayed for each group. The total for all groups may also be displayed.

A data value may be selected in order to display detailed data for superspells that have been grouped to produce the data value. Alternatively, the detailed data may also be reached using a tab in the top frame 24 or links within the CUSUM display pages. The detailed data may include information on the date of admission or main procedure, the diagnosis or procedure codes, details of the trust, hospital, consultant team and PCT responsible for treating the patient. The data may also include patient details such as their age, gender, deprivation, country of origin or details of the treatment outcomes such as the length of stay (both including and excluding transfers), where and when the patient was discharged, the number of episodes and number of spells.

Links to the details of any post-transfer spells and the entire patient history for the patient associated with each superspell may also be provided.

**The User Interface**

Access to the data analysis system may be controlled by a username and password login. Each user account is allocated an appropriate level of access. For example, a trust, such as a group of hospitals, is given a user account with access to all the data for the trust along with the ability to create, edit and delete any users that will access the data through the user account.

The unit administrator is able to control the access level of users they have created. For example, a user may be limited to viewing data for a single consultant team, speciality or hospital. Alternatively, they could be allowed to view data at a trust or multiple trust level. The hospital sites and consultant teams may also be grouped into locally-relevant aggregations. Ordinary users may also only be able to change their password.

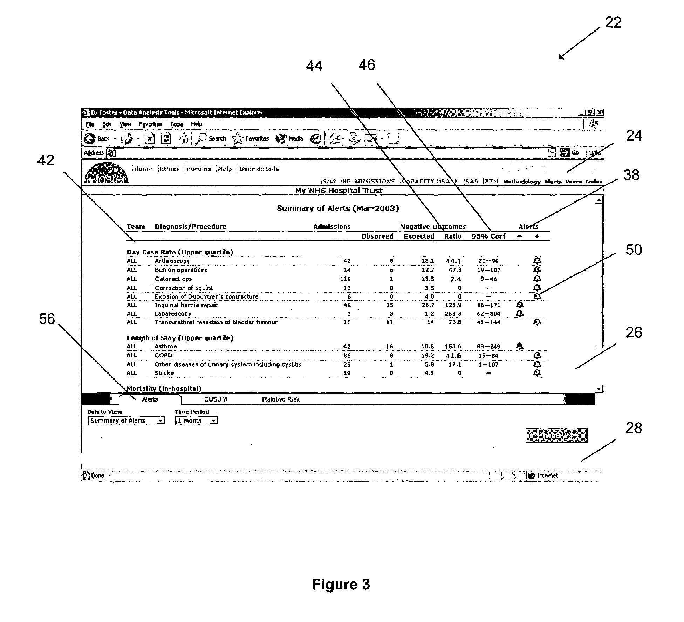

Interaction with the system is carried out through a user interface 22. Preferably the user interface comprises standard HTML pages presented to the user via a Web browser and appears as three frames within a single window as shown in Figures 2, 3, 4a and 4b.

The top frame 24 preferably, contains a menu of options. The menu allows users to view pages, enabling account administration, providing information on the CUSUM methodology, showing how the various diagnosis and procedure groups are constituted, for setting up peer trusts for use in comparative analyses (see Relative Risk below) or displaying a glossary of terms used throughout the system.

The bottom frame 28 preferably contains a number of tabs each relating to an analysis. The analysis may be one of "Alerts", "CUSUM" or "Relative Risk"; however, other analyses are possible. When selected, each tab 56 acts to reveal the options, such as criteria 30, which may be selected when performing the analysis associated with the tab 56. Preferably, the options are presented in the form of a dropdown menu and when an option is selected the relevant heading is displayed above the menu.

There may also be a button which, when selected causes the appropriate analysis to be generated based on the options selected.

The middle frame 26 contains the result when an item from the top frame 24 is selected or the button is clicked in the bottom frame 28. This will be a graph, a chart or a table and a summary that confirms the options selected by the user.

There is also an option to switch between a graph and a table view of the data, and a button to display a printable view of both the graph and the table.

**Claims**

1. A method for analysing data comprising the steps of:

   receiving patient data;
   receiving criteria representative of one or more selected characteristic the patient data to be analysed;

filtering the patient data according to the criteria; and

calculating a representation of the filtered data.

2. A method for analysing data as claimed in Claim 1 wherein the calculation produces a control chart.

3. A method for analysing data as claimed in Claim 1 or Claim 2 wherein the control chart is calculated using patient data that is weighted according to the patient outcome.

4. A method for analysing data as claimed in Claim 3 wherein the weighted patient data is calculated using the following formulae:

$$W_t = \log \frac{(1-p_t+R_0p_t)R_A}{(1-p_t+R_Ap_t)R_o}$$

if the outcome is negative
and

$$W_t = \log \frac{1-p_t+R_0p_t}{1-p_t+R_Ap_t}$$

if the outcome is positive

where $R_0$ corresponds to the existing benchmark;

$R_A$ corresponds to a change in performance where the performance has diverged significantly from the mean;

$y_t$ is the actual patient outcome;

and $p_t$ is the estimated risk.

5. A method for analysing data as claimed in Claims 2 to 4 wherein the control chart produced is a cumulative sum chart representation.

6. A method for analysing data as claimed in Claim 5 wherein the cumulative sum chart is a positive cumulative sum chart.

7. A method for analysing data as claimed in Claim 6 wherein the positive cumulative sum chart is calculated using the following formula:

$$X_t = \min(0, X_{t-1} - W_t)$$

where $X_t$, is the current CUSUM statistic value, $W_t$ is the patient weighting and t is equal to the number of patients, including the patient currently being analysed, whose data has been analysed.

8. A method for analysing data as claimed in Claim 5 wherein the control chart is a negative cumulative sum chart.

9. A method for analysing data as claimed in Claim 8 wherein the negative cumulative sum chart is calculated using the following formula:

$$X_t = \max(0, X_{t-1} + W_t), t=1, 2, 3...$$

where $X_t$, is the current CUSUM statistic value, $W_t$ is the patient weighting and t is equal to the number of patients, including the patient currently being analysed, whose data has been analysed.

10. A method of analysing data as claimed in any preceding claim further comprising the steps of:

calculating a benchmark value; and
setting a threshold value above which the graph has diverged significantly from the benchmark value.

11. A method of analysing data as claimed in Claim 10 wherein the benchmark value is the mean of the patient data.

12. A method for analysing data as claimed in any preceding claim wherein the data is grouped according to date.

13. A method for analysing data as claimed in any preceding claim wherein the criteria includes the outcome.

14. A method for analysing data as claimed in any preceding claim wherein the criteria includes one of an emergency diagnosis, surgical procedure or the group of the diagnoses resulting in 80% of hospital mortaility.

15. A method for analysing data as claimed in any preceding claim wherein data is automatically filtered and the calculation is automatically performed on receipt of new patient data according to pre-defined criteria.

16. A method for analysing data as claimed in Claim 1 further comprising the steps of:

receiving an option according to which the patient data is to be grouped; and
grouping the patient data according to the analysis option.

17. Apparatus comprising:

a patient data input;
an input for criteria representative of one or more selected characteristic the patient data to be analysed;
filtering means for filtering patient data according to the criteria; and
calculating means for processing filtered data to produce a representation of the filtered data.

**18.** Apparatus comprising:

a patient data input;
an input for criteria representative of one or more selected characteristic the patient data to be analysed; and
a processor arranged to filter patient data according to the criteria and
to process filtered data to produce a representation of the filtered data.

**19.** A server arranged to

receive patient data;
receive criteria representative of one or more selected characteristic the patient data to be analysed from a client;
filter the patient data according to the criteria; and
calculate a representation of the filtered data.

**20.** A client comprising a :

user interface;
user input for inputting criteria representative of one or more selected characteristic the patient data to be analysed;
a server connection;
output for sending the criteria to a server;
server input for receiving a calculated representation of the filtered data; and
means for viewing a representation of the filtered data.

Step
10

| Inputting of criteria defining the patient group to be analysed |
| Inputting of parameters for calculating the control chart statistic |

Filtering of patient data held in a database according to the inputted criteria and parameters

Step
12

Retrieval of superspells for all the patients matching the inputted criteria

Step
14

Calculating the weights for every patient superspell

Step
16

Calculating the CUSUM statistic for every patient superspell

Step
18

Returning a representation of the calculated CUSUM statistic

Step
20

**Figure 1**

Figure 2

Figure 3

Figure 4a

22

24

| Age (7 groups) | Admissions | Observed | % | Expected | % | RRR | Low | High |
|---|---|---|---|---|---|---|---|---|
| ALL | 6078 | 900 | 14.8% | 897.7 | 14.8% | 100.3 | 93.8 | 107.1 |
| 15-44 | 362 | 54 | 14.9% | 53.8 | 14.9% | 100.3 | 75.9 | 131.6 |
| 45-64 | 2530 | 354 | 14% | 351.3 | 13.9% | 100.8 | 90.6 | 111.9 |
| 65-74 | 1845 | 303 | 16.4% | 279.4 | 15.1% | 108.4 | 96.7 | 121.5 |
| 75-84 | 1040 | 140 | 13.5% | 166.4 | 16% | 84.2 | 71 | 99.5 |
| 85+ | 301 | 49 | 16.3% | 46.9 | 15.6% | 104.5 | 77.9 | 139.1 |

54

56

26

30

28

**Figure 4b**